# EUROPÄISCHE PATENTANMELDUNG

(11) **EP 2 138 569 A1**
(43) Veröffentlichungstag der Anmeldung: **30.12.2009**
(21) Anmeldenummer: 09008397.3
(22) Anmeldetag: 26.06.2009
(51) Int. Cl.: C12M 1/107, B01D 53/78

(54) **Verfahren zur Erzeugung eines verbrennungsmotortauglichen Mischgases und Vorrichtung hierfür**

(30) Priorität: 27.06.2008 DE 102008030649; 12.08.2008 EP 08014339
(71) Anmelder: Jacobs, Hans-Hermann, 29643 Neuenkirchen (DE)
(72) Erfinder: Jacobs, Hans-Hermann, 29643 Neuenkirchen (DE)
(74) Vertreter: Walther, Walther & Hinz GbR

(57) **Zusammenfassung**

Gegenstand der Erfindung ist ein Verfahren zur Erzeugung eines verbrennungsmotortauglichen Mischgases, bei dem in einer Biogasanlage fermentierbare Rohstoffe unter Bildung eines Gärsubstrates vergast werden und bei dem in einem Feststoffvergaser Feststoffe, insbesondere Holz oder andere nachwachsende Feststoffe, vergast werden, wobei das dabei entstehende Feststoffgas der Biogasanlage zugeführt wird und eine Vorrichtung hierfür. Ein Verfahren und eine Vorrichtung zu schaffen, mit der ein aus Feststoffen erzeugtes Feststoffgas zur Verwendung in einem Verbrennungsmotor nutzbar gemacht werden kann, ohne dass dabei der Biogasprozess gestört wird, wird dadurch erreicht, dass der Biogsanlage ein Teil des Gärsubstrates entnommen wird, dass diesem entnommenen Gärsubstrat das Feststoffgas zugeführt wird, und dass dieses Gemisch, insbesondere zusammen mit dem zwischenzeitlich ausgetretenen Mischgas, zur weiteren Vergasung in die Biogasanlage eingeleitet wird.

## Beschreibung

Zur Gewinnung umweltfreundlicher Energie wird zunehmend angestrebt, Holz zu vergasen und das daraus entstehende Holzgas in Verbrennungsmotoren weiterzuverarbeiten, um somit thermische und elektrische Energie zu erhalten. Allerdings befinden sich im Holzgas Verunreinigungen, wie zum Beispiel Teer oder andere Schadstoffe, die mit der Zeit Teile des Verbrennungsmotors beschädigen, und somit hohe Reparaturkosten verursachen. Deshalb wird angestrebt, Holzgas oder andere aus organischen Rohstoffen erzeugte Gase, derart aufzubereiten, dass die für den Verbrennungsmotor schädlichen Verunreinigungen beseitigt werden.

Hierzu wird in der DE 102 45 469 A1 und in der EP 1 637 585 A1 vorgeschlagen, das beispielsweise mittels Pyrolyse erzeugte Feststoffgas einer Biogasanlage zuzuführen. In der EP 1 637 585 A1 ist weiterhin ausgeführt, dass das Feststoffgas mittels eines Druckgebläses in die Biogasanlage eingeführt wird, wobei das Feststoffgas ins Innere des Gärsubstrats eingeleitet wird. Dabei hat sich aber herausgestellt, dass beim Einleiten des Feststoffgases in die Biogasanlage der eigentliche Biogasprozess deutlich gestört wird, insbesondere dadurch, dass das druckbehaftete Einblasen des Feststoffgases das Gärsubstrat aufgewühlt wird. Auch hat sich herausgestellt, dass hierdurch keine ausreichende Reinigung des Feststoffgases erfolgt, sondern dass lediglich ein Teil der Schadstoffe abgebaut wird, und dass die verbleibenden Schadstoffe durch das Biogas verdünnt werden. Ein solches Mischgas hat zwar weniger Schadstoffe, jedoch greifen die verbleibenden Schadstoffe nach wie vor Dichtungen und andere Bauteile des Verbrennungsmotors an.

Darüber hinaus ist in der EP 1 637 585 A1 vorgeschlagen, dass die Vermischung des Feststoffgases mit dem Güllesubstrat in einem gesonderten Behälter außerhalb der Biogasanlage durchgeführt werden kann, so dass die dann entstandene Mischung anschließend in den Fermenter eingespeist wird. In der Praxis hat sich jedoch gezeigt, dass beim externen Mischen des Feststoffgases mit dem Gärsubstrat auch Gase frei werden, die wieder mittels Druckgebläse in die Biogasanlage eingebracht werden, so dass sie oben genannten Nachteile wieder auftreten. Außerdem kann hierbei ein Teil des Mischgases verloren gehen, so dass die Effektivität des Systems verschlechtert wird.

Davon ausgehend liegt der vorliegenden Erfindung die Aufgabe zu Grunde, ein Verfahren und eine Vorrichtung der eingangs genannten Art zu schaffen, mit dem/der ein aus Feststoffen erzeugtes Feststoffgas zur Verwendung in einem Verbrennungsmotor nutzbar gemacht werden kann, ohne dass dabei der Biogasprozess gestört wird.

Als technische Lösung dieser Aufgabe wird erfindungsgemäß ein Verfahren mit den Merkmalen des Anspruches 1 und eine Vorrichtung mit den Merkmalen des Anspruches 10 vorgeschlagen. Vorteilhafte Weiterbildungen dieses Verfahrens und dieser Vorrichtung sind den jeweiligen Unteransprüchen zu entnehmen.

Ein nach dieser technischen Lehre ausgeführtes Verfahren und eine nach dieser technischen Lehre ausgebildete Vorrichtung haben den Vorteil, dass erfindungsgemäß der Vermischungsprozess von Feststoffgas und Gärsubstrat außerhalb der eigentlichen Biogasanlage erfolgt, wobei nur ein geringer Teil des Gärsubstrats der Biogasanlage entnommen wird. Hierdurch wird erreicht, dass die Biogasanlage voll funktionstüchtig bleibt, und den Biogasprozess quasi unverändert fortsetzen kann, so dass hier keinerlei Nachteile zu befürchten sind. Ein weiterer Vorteil besteht darin, dass das während des Einführens des Feststoffgases in das Gärsubstrat austretende Mischgas ebenfalls wieder in die Biogasanlage eingeleitet wird.

Dabei hat es sich als vorteilhaft erwiesen, diesen gesamten Prozess kontinuierlich durchzuführen, dass heißt, kontinuierlich Gärsubstrat aus der Biogasanlage zu entnehmen, mit dem Feststoffgas zu mischen und sowohl das Gemisch, als auch das zwischenzeitlich ausgetretene Mischgas zurück in die Biogasanlage zu leiten. Durch einen solchen kontinuierlichen Prozess wird der Gärprozess innerhalb der eigentlichen Biogasanlage nicht wesentlich gestört, so dass die Ausbeute der Biogasanlage unverändert bleibt. Gleichzeitig ist es mit einer solchen kontinuierlichen Zumischung möglich, auch das kontinuierlich anfallende Feststoffgas zeitnah zu verarbeiten.

In einer bevorzugten Ausführungsform wird das extern erzeugte Gemisch aus Feststoffgas und Gärsubstrat zusammen mit dem zwischenzeitlich ausgetreten Mischgas nahezu drucklos in die Biogasanlage eingeleitet. Hierzu reicht ein Überdruck von 5 bis 20 mbar , vorzugsweise 10 mbar aus, was technisch als drucklos gilt. Hierdurch wird erreicht, dass sowohl das Gemisch als auch das Mischgas sanft in die Biogasanlage eingeleitet werden, ohne dort Verwirbelungen, Blasen oder sonstige Veränderungen hervorzurufen. Dies trägt erheblich zum effektiven Betrieb der Biogasanlage bei.

In einer vorteilhaften Ausführungsform wird das Feststoffgas vor dem Zuführen zum Gärsubstrat unter den Taupunkt der Schadstoffe gekühlt. Hierdurch sondern sich bereits einige Schadstoffe ab und können somit besser ausgeschieden werden, insbesondere wenn das Feststoffgas in einer Flüssigkeitsvorlage gekühlt oder gereinigt wird.

In anderen, vorteilhaften Ausführungsformen wird das Feststoffgas ins Innere der Flüssigkeit der Flüssigkeitsvorlage geführt, so dass neben der Abkühlung des Feststoffgases auch bereits eine gewisse Reinigung erfolgt, denn hierbei werden bereits einige Schadstoffe im Wasser gebunden.

Die Zuführung des Feststoffgases in das Gärsubstrat erfolgt wiederum mit einem Druckgebläse, wobei das Druckgebläse so eingestellt ist, dass der in der Gärsubstratvorlage entstehende Überdruck 20 mbar nicht überschreitet.

Ein solches Verfahren wird vorteilhafter Weise in einer Vorrichtung umfassend eine Biogasanlage und eine Gärsubstratvorlage durchgeführt, wobei die Gärsubstratvorlage über eine Zu- und eine Ableitung mit der Biogasanlage verbunden ist, wobei die Zuleitung unterhalb der Füllstandshöhe der Biogasanlage in diese mündet. Hierdurch ist sichergestellt, dass stets Gärsubstrat aus dem Fermenter in die Gärsubstratvorlage gelangt, während auf der anderen Seite das Gemisch aus Feststoff und Gärsubstrat vorzugsweise oberhalb der Füllstandshöhe in den Fermenter eingeleitet wird, so dass dieses Gemisch ohne große Bewegungen zu erzeugen dem Fermenter zugeführt werden kann. Gleichzeitig wird hierdurch erreicht, dass das in der Gärsubstratvorlage anfallende Mischgas unmittelbar mit dem Biogas vermischt werden kann, ohne den eigentlichen Biogasprozess zu stören.

Weitere Vorteile des erfindungsgemäßen Verfahrens und der erfindungsgemäßen Vorrichtung ergeben sich aus der beigefügten Zeichnung und den nachstehend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Die Zeichnung zeigt eine schematische Darstellung der erfindungsgemäßen Vorrichtung.

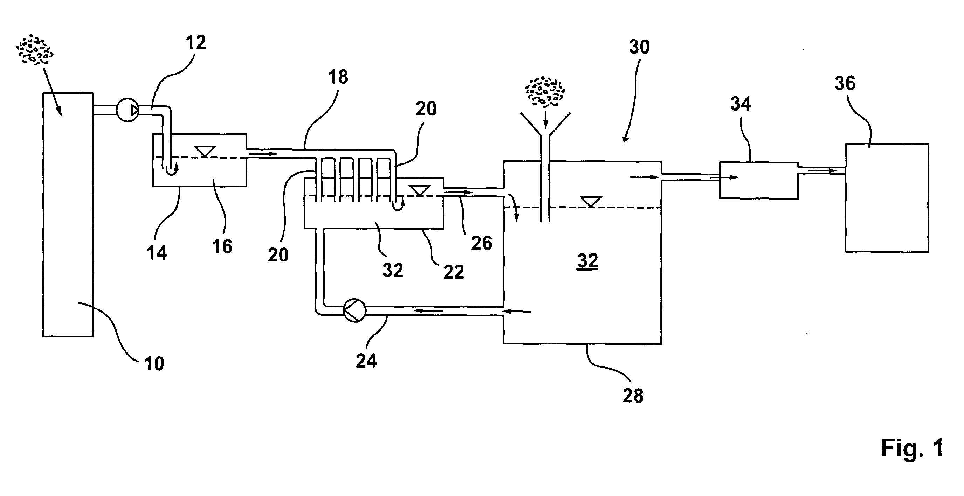

In der Figur 1 ist in schematischer Darstellung eine erfindungsgemäße Vorrichtung zur Erzeugung eines verbrennungsmotortauglichen Mischgases dargestellt. Diese Vorrichtung umfasst einen Feststoffvergaser 10, insbesondere einen Holzvergaser, in dem organische Feststoffe, wie beispielsweise Holz oder Kohle, mittels Pyrolyse vergast werden. Das dabei entstehende Feststoffgas wird über eine Gasleitung 12 in eine Flüssigkeitsvorlage 14 geleitet, die teilweise mit Wasser 16 gefüllt ist, wobei die Gasleitung 12 so weit nach unten geführt wird, dass diese in das Wasser 16 mündet. Abgangsseitig ist an der Flüssigkeitsvorlage 14 eine Ausleitung 18 angebracht, die mehrere Endstücke 20 aufweist.

An die Gasleitung 12 angeschlossen ist ein Gebläse 19, welches das aus dem Feststoffvergaser 10 kommende Feststoffgas in die Flüssigkeitsvorlage 14, insbesondere in das Wasser 16 in der Flüssigkeitsvorlage 14, befördert. Das Wasser 16 der Flüssigkeitsvorlage 14 nimmt dabei einen Teil der Verunreinigungen des Feststoffgases auf. In die Flüssigkeitsvorlage 14 wird kontinuierlich oder in vorgegebenen Abständen Wasser 16 zugeführt, wobei überschüssiges und gegebenenfalls verunreinigtes Wasser 16 über die Ausleitung 18 zusammen mit dem gereinigten Feststoffgas in eine Gärsubstratvorlage 22 abgeführt wird.

In einer hier nicht dargestellten Ausführungsform wird das Wasser der Flüssigkeitsvorlage regelmäßig ausgetauscht und/oder ergänzt. Das durch die Feststoffgase verunreinigte Wasser wird dann dem Biogasprozess zugeführt, wobei die Schadstoffe zumindest teilweise abgebaut werden.

Die Ausleitung 18 weist in der hier dargestellten Ausführungsform fünf Endstücke 20 auf, die in die Gärsubstratvorlage 22 münden. Diese Gärsubstratvorlage 22 ist mit einer Zuleitung 24 und mit einer Ableitung 26 mit dem Fermenter 28 an der Biogasanlage 30 verbunden. Dabei wird über eine Pumpe 31 kontinuierlich Gärsubstrat 32 aus dem Fermenter 28 über die Zuleitung 24 in die Gärsubstratvorlage 22 gepumpt. Gleichzeitig gelangt auf der anderen Seite Gärsubstrat 32 über die Ableitung 26 zurück in den Fermenter 28, wobei die Ableitung 26 oberhalb der Füllstandshöhe des Fermenters 28 mündet, während die Zuleitung 24 unterhalb der Füllstandshöhe des Fermenters 28 ansetzt. Die Endstücke 20 der Ausleitung 18 reichen bis in das Innere des Gärsubstrats 32 hinein, so dass das Feststoffgas in das Innere des Gärsubstrats 32 hineintransportiert wird.

Im Fermenter 28 herrscht kein nennenswerter Überdruck, das heißt maximal 10 mbar. Damit das sich in der Gärsubstratvorlage 22 bildende Mischgas in den Fermenter 28 gelangen kann, muss hier ein ebenfalls leichter, wenn auch minimaler, Überdruck von ebenfalls 5 - 10 mbar vorherrschen. Ein solcher minimaler Überdruck von 5 mbar, höchstens 10 mbar, wird in der Biogasbranche als drucklos angesehen.

Damit nun das Feststoffgas aus dem Feststoffvergaser 10 in die Flüssigkeitsvorlage 14 gelangen kann, wird dieses mit dem Gebläse 19 mittels Überdruck in das Wasser 16 der Flüssigkeitsvorlage 14 eingeblasen. Dabei ist der Druck so eingestellt, dass in der Flüssigkeitsvorlage 14 solch ein Überdruck vorherrscht, dass das gekühlte und gereinigte Feststoffgas über die Ausleitung 18 noch in das Gärsubstrat 32 gedrückt werden kann, wobei aber der Überdruck in der Gärsubstratvorlage etwa 10 mbar, aber nicht mehr als 20 mbar betragen soll. In Ausnahmefällen kann dies auch leicht überschritten werden.

Das derart erzeugte Mischgas wird dann vom Fermenter 28 in eine aus dem Stand der Technik bekannte Kühlungs- und Reinigungsvorrichtung 34 geleitet, bevor es in einem Verbrennungsmotor 36 weiterverwendet wird.

Nachfolgend wird das Verfahren zur Erzeugung eines verbrennungsmotortauglichen Mischgases anhand der in Figur 1 abgebildeten Vorrichtung näher erläutert:

Zunächst wird aus festen, organischen Brennstoffen, wie zum Beispiel Holz, Kohle oder dergleichen mittels Pyrolyse im Feststoffvergaser 10 ein Feststoffgas erzeugt. Dieses Feststoffgas wird dann über das Gebläse 19 in der Gasleitung 12 in das Wasser 16 einer Flüssigkeitsvorlage 14 geleitet. Dabei wird das Feststoffgas gekühlt und einige dabei auskondensierende Schadstoffe werden im Wasser 16 chemisch gebunden oder mechanisch gehalten. Dabei erfolgt eine Kühlung des Feststoffgases bis unterhalb der Taupunkte der jeweiligen Schadstoffe.

Das derartig vorgereinigte und gekühlte Feststoffgas wird dann über die Ausleitung 18 und die verschiedenen Endstücke 20 mittels Überdruck in eine Gärsubstratvorlage 22 geleitet, wobei das Feststoffgas bis ins Innere des in der Gärsubstratvorlage 22 befindlichen Gärsubstrats 32 gedrückt wird. Außerdem wird der Flüssigkeitsvorlage 14 frisches Wasser 16 zugeführt, so dass ein Teil des verunreinigten Wassers 16 über die Ableitung 18 in das Gärsubstrat 32 gelangt.

Im Gärsubstrat 32 werden organischen Stoffe aus dem Feststoffgas und aus dem verunreinigten Wasser 16 gebunden. Das derart zumindest teilweise gereinigte Feststoffgas steigt dann aus dem Gärsubstrat 32 auf und vermischt sich mit dem in der Gärsubstratvorlage 22 befindlichen Biogas. Dieses Mischgas, welches sich aus den Feststoffgas und dem im Gärprozess entstehenden Biogas zusammensetzt wird dann über die Ableitung 26 in den Fermenter 28 einer Biogasanlage 30 geführt. Dabei ist die Ableitung 26 so angeordnet, dass das Mischgas direkt dem im Fermenter 28 befindlichen Biogas zugeführt wird, ohne nochmals mit dem Gärsubstrat 32 vermischt zu werden.

Dieser ganze Prozess läuft kontinuierlich ab, das heißt, über die Pumpe 31 wird kontinuierlich Gärsubstrat 32 aus dem Fermenter 28 über die Zuleitung 24 in die Gärsubstratvorlage 22 gepumpt, während gleichzeitig ebenfalls kontinuierlich Feststoffgas aus dem Feststoffvergaser 10 und in gewissem Maße auch verunreinigtes Wasser 16 in das Gärsubstrat 32 eingeleitet wird. Über die Ableitung 26 wird dann das Gärsubstrat 32 zusammen mit dem Mischgas dem Fermenter 28 zugeführt, wobei sich das Mischgas mit dem Biogas vermischt, und wobei das Gärsubstrat 32 nach unten in den Fermenter 28 fällt. Die in dem Gärsubstrat 32 befindlichen, aus dem Feststoffgas stammenden Verunreinigungen werden im Fermenter 28 zumindest teilweise biologisch abgebaut.

Aus dem Fermenter 28 der Biogasanlage 30 wird das hierdurch entstandene Mischgas dann in allgemein bekannter Weise einer Kühlungs- und Reinigungsvorrichtung 34 zugeführt, bevor es in einem Verbrennungsmotor 36 verarbeitet wird.

Dabei hat es sich als vorteilhaft erwiesen, bei einer zu fördernden Menge von 200 m³ je Stunde Gärsubstrats 32 etwa 120 bis 300 m³ Feststoffgas zuzuführen, um eine stabile Verarbeitung zu erreichen. In Extremfällen kann auf 1 m³ Feststoffgas auch nur 0,1 m³ Gärsubstrat kommen, ohne dass die Wirtschaftlichkeit der Anlage beeinträchtigt wird.

Erfahrungsgemäß hat sich gezeigt, dass das Gebläse zum Transport des Feststoffgases in der Gasleitung 12 einen Überdruck von 40 bis 100 mbar erzeugen muss, damit das Mischgas und das Gärsubstrat 32 noch quasi drucklos in den Fermenter 28 gelangen kann.

### Bezugszeichenliste:

- 10: Feststoffvergaser
- 12: Gasleitung
- 14: Flüssigkeitsvorlage
- 16: Wasser
- 18: Ausleitung
- 19: Gebläse
- 20: Endstücke
- 22: Gärsubstratvorlage
- 24: Zuleitung
- 26: Ableitung
- 28: Fermenter
- 30: Biogasanlage
- 31: Pumpe
- 32: Gärsubstrat
- 34: Kühlungs- und Reinigungsvorrichtung
- 36: Verbrennungsmotor

## Patentansprüche

1. Verfahren zur Erzeugung eines verbrennungsmotortauglichen Mischgases, bei dem in einer Biogasanlage (30) fermentierbare Rohstoffe unter Bildung eines Gärsubstrates (32) vergast werden und bei dem in einem Feststoffvergaser (10) Feststoffe, insbesondere Holz, Kohle oder nachwachsende Feststoffe, vergast werden, wobei das dabei entstehende Feststoffgas der Biogasanlage (30) zugeführt wird,
**dadurch gekennzeichnet,**
**dass** der Biogsanlage (30) ein Teil des Gärsubstrates (32) entnommen wird, dass diesem entnommenen Gärsubstrat (32) das Feststoffgas zugeführt wird, und dass dieses Gemisch zusammen mit dem zwischenzeitlich ausgetretenen Mischgas, zur weiteren Vergasung in die Biogasanlage (30) eingeleitet wird.

2. Verfahren nach Anspruch 1,
**dadurch gekennzeichnet,**
**dass** der Biogasanlage (30) kontinuierlich Gärsubstrat (32) entnommen wird und dass das Gemisch aus Gärsubstrat (32) und Feststoffgas, insbesondere zusammen mit dem zwischenzeitlich ausgetretenen Mischgas, kontinuierlich der Biogasanlage (30) zugeführt wird.

3. Verfahren nach einem der vorangehenden Ansprüche,
**dadurch gekennzeichnet,**
**dass** das Gemisch, insbesondere zusammen mit dem zwischenzeitlich ausgetretenen Mischgas, nahezu drucklos in die Biogasanlage (30) eingeleitet wird.

4. Verfahren nach einem der vorangehenden Ansprüche,
**dadurch gekennzeichnet,**
**dass** das Feststoffgas vor dem Zuführen zum Gärsubstrat (32) unter den Taupunkt der Schadstoffe abgekühlt wird.

5. Verfahren nach Anspruch 4,
**dadurch gekennzeichnet,**
**dass** das Feststoffgas in einer Flüssigkeitsvorlage (14), insbesondere einer Wasservorlage, abgekühlt oder gereinigt wird.

6. Verfahren nach Anspruch 5,
**dadurch gekennzeichnet,**
**dass** das Feststoffgas ins Innere der Flüssigkeit (16) eingeleitet wird.

7. Verfahren nach einem der Ansprüche 5 oder 6,
**dadurch gekennzeichnet,**
**dass** das Feststoffgas dem aus der Biogasanlage (30) entnommenen Gärsubstrat (32) zugeführt wird, indem aus der Flüssigkeitsvorlage (14) austretendes Feststoffgas in das Innere des Gärsubstrates (32) geleitet wird.

8. Verfahren nach einem der vorangehenden Ansprüche,
**dadurch gekennzeichnet,**
**dass** das Feststoffgas mittels Überdruck in dass Innere des in einer Gärsubstratvorlage (22) befindlichen Gärsubstrates (32) eingeführt wird, wobei der Überdruck in der Gärsubstratvorlage (22) 15 mbar nicht überschreiten soll.

9. Verfahren nach einem der vorangehenden Ansprüche,
**dadurch gekennzeichnet,**
**dass** die Feststoffe im Vergaser (10) mittels Pyrolyse vergast werden.

10. Vorrichtung zur Erzeugung eines verbrennungsmotortauglichen Mischgases, mit einer Biogasanlage (30) zur Erzeugung eines Gases aus fermentierbaren Rohstoffen unter Bildung eines Gärsubstrates (32) und mit einem Feststoffvergaser (10) zur Vergasung von Feststoffen, insbesondere mittels Pyrolyse,
**gekennzeichnet durch,**
eine außerhalb der Biogasanlage (30) vorgesehene Gärsubstratvorlage (22) zum Einleiten des Feststoffgases in das Gärsubstrat (32), wobei die Gärsubstratvorlage (22) über eine Zuleitung (24) und eine Ableitung (26) mit der Biogasanlage (30) verbunden ist, wobei die Zuleitung (24) unterhalb der Füllstandshöhe der Biogasanlage (30) in diese mündet.

11. Vorrichtung nach Anspruch 10,
**gekennzeichnet durch**
eine Flüssigkeitsvorlage (14), insbesondere eine Wasservorlage, zur Reinigung und/oder Abkühlung des Feststoffgases.

12. Vorrichtung nach Anspruch 11,
**dadurch gekennzeichnet,**
**dass** eine vom Feststoffvergaser (10) kommende Gasleitung (12) bis in das Innere der Flüssigkeit (16) der Flüssigkeitsvorlage (14) reicht.

13. Vorrichtung nach einem der Ansprüche 11 oder 12,
**dadurch gekennzeichnet,**
**dass** die Flüssigkeitsvorlage (14) eine Ausleitung (18) aufweist, die bis in die Gärsubstratvorlage (22), insbesondere bis ins Innere des Gärsubstrates (32) reicht.

14. Vorrichtung nach Anspruch 13,
**dadurch gekennzeichnet,**
**dass** die Ausleitung (18) mehrere Endstücke (29) aufweist.
